(19) Europäisches Patentamt / European Patent Office / Office européen des brevets

(11) **EP 2 203 114 B1**

(12) **EUROPÄISCHE PATENTSCHRIFT**

(45) Veröffentlichungstag und Bekanntmachung des Hinweises auf die Patenterteilung:
**16.11.2011 Patentblatt 2011/46**

(51) Int Cl.:
***A61B 5/00*** *(2006.01)*

(21) Anmeldenummer: **08801910.4**

(22) Anmeldetag: **08.09.2008**

(86) Internationale Anmeldenummer:
**PCT/EP2008/007331**

(87) Internationale Veröffentlichungsnummer:
**WO 2009/033625 (19.03.2009 Gazette 2009/12)**

(54) **MEDIZINISCHE MESSVORRICHTUNG ZUR BIOELEKTRISCHEN IMPEDANZMESSUNG**

MEDICAL MEASURING DEVICE FOR BIOELECTRICAL IMPEDANCE MEASUREMENT

DISPOSITIF DE MESURE MEDICAL POUR MESURE D'IMPEDANCE BIOELECTRIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **07.09.2007 DE 102007042550**

(43) Veröffentlichungstag der Anmeldung:
**07.07.2010 Patentblatt 2010/27**

(73) Patentinhaber: **Flore, Ingo**
**44141 Dortmund (DE)**

(72) Erfinder:
• **CHO, Ok, Kyung**
  **58239 Schwerte (DE)**
• **KIM, Yoon, Ok**
  **58239 Schwerte (DE)**

(74) Vertreter: **Isfort, Olaf**
**Schneiders & Behrendt**
**Rechtsanwälte - Patentanwälte**
**Huestrasse 23**
**44787 Bochum (DE)**

(56) Entgegenhaltungen:
**WO-A-2005/077260      WO-A-2007/017266**
**US-A1- 2005 014 999**

**Beschreibung**

Die Erfindung betrifft eine medizinische Messvorrichtung mit einer Impedanz-messeinheit zur Erfassung eines Impedanzmesssignals von der Hautoberfläche eines zu untersuchenden Patienten über wenigstens ein Messelektrodenpaar.

Die zur Zeit benutzen bioelektrischen Impedanzverfahren werden bei der Bestimmung des Ernährungszustandes sowohl bei gesunden als auch bei kranken Menschen eingesetzt.

Die direkte Messung der Körperzusammensetzung ist zur Zeit nur in Forschungseinrichtungen durch Wiegen des Patienten unter Wasser möglich. Beim so genannten hydrostatischen Wiegen wird ausgenutzt, dass Fett, Muskeln, Knochen, Körperflüssigkeiten und andere Körperbestandteile unterschiedliche spezifische Dichten besitzen. Da dieses Messverfahren sehr aufwändig und unangenehm für den Patienten ist, werden häufig andere, indirekte Verfahren zur Bestimmung der Körperzusammensetzung benutzt. Eine weit verbreitete indirekte Methode ist die Messung der bioelektrischen Impedanz des Körpers. Bei dieser Technik wird ausgenutzt, dass die Impedanz des menschlichen Körpers mit den unterschiedlichen Leitfähigkeiten innerhalb des Körpers zusammenhängt. Wird in biologischen Strukturen ein konstanter Wechselstrom kleiner Amplitude angelegt, so kann eine frequenzabhängige Impedanz gemessen werden. Der menschliche Körper besteht aus intra- und extrazellulärer Flüssigkeit, welche als elektrische Leiter angesehen werden können, und Zellmembranen, welche einen kapazitiven Charakter aufweisen. Bei kleinen Frequenzen um 1 kHz geschieht der Stromfluss hauptsächlich durch die extrazelluläre Flüssigkeit, da die Zellmembranen aufgrund ihres kapazitiven Charakters als Kondensator wirken. Bei größeren Frequenzen wird der Strom auch über die Zellmembranen und die interzelluläre Flüssigkeit geleitet. Im Bereich großer Frequenzen geschieht der Stromfluss also sowohl ohmsch durch die extrazelluläre Flüssigkeit als auch kapazitiv durch die Zellmembranen und die interzelluläre Flüssigkeit. Der resultierende, messbare Wechselstrom-Widerstand besitzt damit einen ohmschen R (Resistanz) und einen kapazitiven $X_c$ Anteil (Reaktanz) Anteil, und man spricht von der Messung der bioelektrischen Impedanz Z. Seit langem wird versucht, aufgrund der gemessenen Resistanz und Reaktanz zusammen mit dem Alter, der Körperlänge und des Geschlechts der untersuchten Person die Körperzusammensetzung zu schätzen und zu bewerten. Dies ist mit Hilfe von zahlreichen Annahmen möglich: Im menschlichen Körper fließt der größte Teil des angelegten Stromes durch die im Körper enthaltene Flüssigkeit. Unter der Annahme, dass die Körpermasse zu exakt 73,2% aus Wasser besteht, lässt sich dann aus dem "gemessenen" Gesamtkörperwasser (TBW) die fettfreie Masse (FFM) abschätzen. Ist die fettfreie Masse bekannt, so kann recht einfach die Fettmasse (FM) aus der Gesamtmasse (GM) des Patienten bestimmt werden.

Diese bekannten und etablierten Techniken bestimmen somit einen Globalindex für den untersuchten Patienten. Dieser Globalindex ist das Resultat des Integralmessverfahrens, welches bei diesen Techniken benutzt wird: Typischerweise werden die Elektroden an beiden Händen angeschlossen (Bestimmung des Globalindexes des Oberköpers) oder es wird eine Messung zwischen einer Hand und einem Fuß durchgeführt (Globalindex für die vermessene Körperseite).

Der Erfindung liegt die Aufgabe zugrunde, eine weiterentwickelte Messvorrichtung zur bioelektrischen Impedanzmessung bereit zu stellen.

Diese Aufgabe löst die Erfindung durch eine Messvorrichtung gemäß Anspruch 1.

Die erfindungsgemäße Messvorrichtung basiert auf einer Weiterentwicklung der etablierten Techniken in Richtung lokaler bioelektrischer Impedanzmessung. Durch eine Verkürzung des Elektrodenabstandes auf den Bereich von weniger als einen Millimeter bis zu einigen Zentimetern wird nicht über den gesamten Körper integriert, sondern die bioelektrische Impedanz lokal aufgenommen. Gemäß der Erfindung berühren sämtliche Elektroden ein und den selben lokalen Bereich der Hautoberfläche, d.h., dass sämtliche Elektroden mit dem selben Körperteil (z.B. Hand, Finger, Fuß oder Zeh) von dem zu untersuchenden Patienten berührt werden.

Zweckmäßig weist die erfindungsgemäße Messvorrichtung zur Messung der lokalen Resistanz und Reaktanz ein Einspeiseelektrodenpaar zur Aufprägung eines Wechselstroms variabler Frequenz über die Hautoberfläche in das Körpergewebe des zu untersuchenden Patienten auf, und zwar in dem die Messelektroden berührenden Bereich der Hautoberfläche.

Vorzugsweise beträgt der Abstand der Einspeiseelektroden, entsprechend den Messelektroden wenige Millimeter bis einige Zentimeter. Als besonders vorteilhaft hat sich eine Ausgestaltung erwiesen, bei welcher die Mess- und Einspeiseelektroden als parallel zueinander verlaufende Kontaktstreifen ausgebildet sind. Dies ermöglicht es, die lokale Impedanz des Körpergewebes ohne verfälschende Einflüsse, z.B. bedingt durch die Übergangswiderstände zwischen Elektroden und Hautoberfläche, zu bestimmen.

Zur Erzeugung des Wechselstrom variabler Frequenz weist die erfindungsgemäße Messvorrichtung zweckmäßig einen Wechselstromgenerator auf. Das Impedanzmesssignal wird durch einen Analog-/Digitalwandler digitalisiert und danach einer diskreten Fouriertransformation (DFT) unterzogen. Der DFT-Algorithmus liefert dann den Real- und den Imaginärteil der Impedanz, d.h. den Resistanz- und den Reaktanzwert. Diese Werte können zur Auswertung digital weiter verarbeitet werden.

Bevorzugt beträgt der Elektrodenabstand bis zu maximal 10 cm, besonders bevorzugt, 50 Mikrometer bis 5 cm, weiter bevorzugt 100 Mikrometer bis 1 cm, höchst bevorzugt 1 mm bis 5 mm.

Durch die Ausgestaltung der erfindungsgemäßen Messvorrichtung ist es möglich, lokale zeitliche Änderungen der Impedanz zu bestimmen. Hierzu weist die Messvorrichtung zweckmäßig eine mit der Impedanzmesseinheit verbundene Auswertungseinheit auf. Die Auswertungseinheit kann programmgesteuert sein, so dass die Auswertung der Impedanzmesssignale flexibel durch Software realisiert werden kann.

Z.B. ändert sich die lokale Bioimpedanz aufgrund der sich ändernden Blutmenge innerhalb eines Pulsschlages, wodurch eine Bestimmung der Herzfrequenz über die lokale bioelektrische Impedanz möglich ist. Als wichtiger physiologischer Parameter wird dabei gleichzeitig die Pulsamplitude bestimmt. Es hat sich gezeigt, dass diese Pulsamplitude mit der Körpertemperatur korreliert, d.h. es ist möglich, mit Hilfe der Bioimpedanzanalyse die Temperatur der untersuchten Körperstelle zu ermitteln. Weiterhin hängt die lokale Bioimpedanz von der Menge der Flüssigkeit, d.h. von der lokalen Blutmenge im untersuchten Gewebe ab, womit es möglich ist, die lokale Durchblutung (die durchblutungsbedingte lokale Volumenschwankung, z.B. in Form eines Volumenpulssignals) des untersuchten Gewebes zu bestimmen. Schließlich ändert sich die lokale bioelektrische Impedanz des Körpers in Abhängigkeit von der Nahrungsaufnahme, so dass mit der Bioimpedanz der Metabolismus untersucht werden kann, welcher bekanntlich vom Blutglukosespiegel bestimmt ist. Die erfindungsgemäße Messvorrichtung ermöglicht somit eine nicht-invasive Überwachung des Blutglukosewertes, wobei die Wirkung der Glukose bzw. der Energiebedarf der durch Glukose initiierten physiologischen Reaktionen im Körper untersucht werden. Durch einen geeigneten Algorithmus, der in der Auswertungseinheit durch Software implementiert ist, ist es möglich, aus den aufgenommen Impedanzmesssignalen Aussagen über den Blutglukosespiegel zu tätigen.

Eine besonders sinnvolle Ausgestaltung der erfindungsgemäßen Messvorrichtung ergibt sich durch eine Kombination mit weiteren Messmodalitäten.

Die erfindungsgemäße Messvorrichtung kann zusätzlich mit einer optischen Messeinheit ausgestattet sein. Diese weist eine Strahlungsquelle zur Bestrahlung des untersuchten Körpergewebes mit elektromagnetischer Strahlung, und wenigstens einen Strahlungssensor zur Detektion der von dem Körpergewebe gestreuten und/oder transmittierten Strahlung auf. Als Strahlungsquelle kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens zwei oder besser drei unterschiedlichen Lichtwellenlängen gemessen wird, um daraus die Sauerstoffkonzentration des Blutes und die Durchblutung des Gewebes zu bestimmen. Die optische Messeinheit bildet somit eine Pulsoximetrieeinheit der erfindungsgemäßen Messvorrichtung.

Gemäß einer sinnvollen Ausgestaltung weist die optische Messeinheit der erfindungsgemäßen Messvorrichtung wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist.

Vorteilhaft ist eine Ausgestaltung der erfindungsgemäßen Messvorrichtung, bei welcher wenigstens zwei Strahlungsquellen vorgesehen sind, welche unterschiedliche Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Messvorrichtung vorteilhafterweise auch eine nicht-invasive Bestimmung des Blutglukosespiegels per optischer Messung. Die redundante Bestimmung des Blutglukosespiegels mittels der verschiedenen Messmodalitäten erhöht die Genauigkeit und Zuverlässigkeit der erfindungsgemäßen Messvorrichtung.

Die zwei Strahlungsquellen der optischen Messeinheit der erfindungsgemäßen Messvorrichtung sollten so ausgelegt sein, dass die von diesen jeweils bestrahlten Volumenbereiche hinsichtlich der Durchblutung mit sauerstoffarmem bzw. sauerstoffreichem Blut unterschiedlich betroffen sind. Dies kann z. B. dadurch erreicht werden, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Gemäß einer sinnvollen Ausgestaltung ist die wenigstens eine Strahlungsquelle mit einem lichtleitenden Element, z.B. einer optischen Faser verbunden. Über das lichtleitende Element wird die von der Strahlungsquelle bzw. den Strahlungsquellen emittierte Strahlung zur Hautoberfläche des zu untersuchenden Patienten geleitet. Es besteht die vorteilhafte Möglichkeit, die Strahlung mehrer Strahlungsquellen, z.B. mehrerer LED-Chips, die auf ein gemeinsames Substrat gebondet sind, in ein einziges lichtleitendes Element einzukoppeln. Dabei können die verschiedenen Strahlungsquellen in unterschiedlicher Weise an das lichtleitende Element gekoppelt sein. Auf diese Weise lässt sich eine unterschiedliche Abstrahlcharakteristik der Strahlung der verschiedenen Quellen in das zu untersuchende Körpergewebe erzielen.

Die Auswertungseinheit der erfindungsgemäßen Messvorrichtung kann mit Vorteil auch zur Auswertung der optischen Messsignale genutzt werden. Hierzu ist die Auswertungseinheit zweckmäßig zur Bestimmung eines lokalen metabolischen Parameters aus der von dem Körpergewebe gestreuten und/oder transmittierten Strahlung der beiden Strahlungsquellen ausgebildet sein. Wenn in dem untersuchten Körpergewebe Sauerstoff verbraucht wird, wird Oxihämoglobin in Desoxihämoglobin umgewandelt. Durch einen Vergleich der aus den unterschiedlichen Volumenbereichen des Körpergewebes stammenden Strahlung der beiden Strahlungsquellen kann die Änderung des Konzentrationsverhältnisses von Oxihämoglobin und Desoxihämoglobin festgestellt werden. Hieraus ergibt sich wiederum der lokale Sauerstoffverbrauch und daraus letztlich (indirekt) der Blutglukosespiegel. Somit ist die Auswertungseinheit der erfindungsgemäßen Messvorrichtung sinnvollerweise eingerichtet zur Bestimmung des lokalen Sauerstoffverbrauchs und/ oder des Blutglukosespiegels anhand der Intensitäten der von dem Körpergewebe gestreuten und/oder transmittierten Strahlung der beiden Strahlungsquellen.

Durch eine EKG-Einheit zur Erfassung eines EKG-Signals über zwei oder mehr EKG-Elektroden wird der Funktionsumfang der erfindungsgemäßen Messvorrichtung vorteilhaft erweitert. Gemäß dieser vorteilhaften Weiterbildung der Erfindung werden mittels der Messvorrichtung z.B. Impedanzmesssignale, pulsoximetrische Signale und EKG-Signale kombiniert erfasst und ausgewertet.

Die Auswertungseinheit der Messvorrichtung kann dann mit Vorteil zur Auswertung des zeitlichen Verlaufs der Volumenpulssignale und der EKG-Signale eingerichtet sein. Mittels einer geeigneten Programmsteuerung ist die Auswertungseinheit der erfindungsgemäßen Messvorrichtung dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund ihrer Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Messvorrichtung erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden.

Es können in die Auswertung der Messsignale auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar. Sinnvollerweise wird wenigstens eine der EKG-Elektroden der erfindungsgemäßen Messvorrichtung gleichzeitig als Einspeise- oder Messelektrode der Impedanzmesseinheit genutzt.

Gemäß einer vorteilhaften Ausgestaltung umfasst die erfindungsgemäße Messvorrichtung einen integrierten Temperatur- oder Wärmesensor. Dieser kann zur Bestimmung der lokalen Wärmeproduktion genutzt werden. Im einfachsten Fall ist der Temperatursensor zur Messung der Oberflächentemperatur der Haut am Messort ausgebildet. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No. 9, S. 2953 bis 2956).

Besonders vorteilhaft ist die erfindungsgemäße Kombination der vorgenannten Messverfahren, nämlich der Impedanzmessung, der Oximetrie, der EKG-Messung und der Temperatur- bzw. Wärmemessung. Mittels der Auswertungseinheit der Messvorrichtung können sämtliche Messsignale durch einen geeigneten Algorithmus ausgewertet und kombiniert werden, um den Metabolismus zu untersuchen. Durch die Kombination der verschiedenen Messmodalitäten wird eine hohe Effektivität und Redundanz und damit Zuverlässigkeit bei der Erkennung von pathologischen Veränderungen erreicht.

Die Kombination der verschiedenen Messmodalitäten, die in der erfindungsgemäßen Messvorrichtung, wie oben beschrieben, zusammengefasst sein können, ist weiterhin vorteilhaft, weil dadurch, wie oben bereits erwähnt, eine nicht-invasive indirekte Messung der Glukosekonzentration möglich ist. Ein mögliches Vorgehen bei der Bestimmung des Blutglukosespiegels mittels der erfindungsgemäßen Vorrichtung wird im Folgenden näher erläutert:

Die erfindungsgemäße Messvorrichtung dient zur Messung und zur Auswertung von Daten, die durch den Stoffwechsel beeinflusst werden. Es leuchtet unmittelbar ein, dass dabei der Energiehaushalt und die Zusammensetzung der von einem Benutzer der Messvorrichtung aufgenommenen Nahrung eine große Rolle spielen. Die Nährstoffe, die am Stoffwechsel beteiligt sind, sind bekanntlich im Wesentlichen Kohlenhydrate, Fette und Eiweiße. Kohlenhydrate werden zur weiteren Verarbeitung in Glukose, Eiweiße in Aminosäuren, und Fette in Fettsäuren umgewandelt. Die Energieträger werden dann wiederum in den Zellen des Körpergewebes zusammen mit Sauerstoff unter Abgabe von Energie zu ATP (Adenosintriphosphorsäure) umgewandelt. ATP ist der eigentliche körpereigene Energieträger. Die Verwendung von Glukose zur Erzeugung von ATP ist bevorzugt. Wenn die Erzeugung von ATP aus Glukose jedoch (z. B. wegen eines Mangels an Insulin) gehemmt ist, findet stattdessen eine verstärkte Fettsäure-Oxidation statt. Der Sauerstoffverbrauch ist bei diesem Prozess allerdings ein anderer.

Die Reaktion des Metabolismus des menschlichen Körpers auf eine Nahrungsaufnahme hängt, wie zuvor erwähnt, von der Zusammensetzung der Nahrung charakteristisch ab. So reagiert beispielsweise das vaskuläre System des Körpers in Abhängigkeit davon, wie viel Energie der Körper zur Verdauung der aufgenommenen Speisen benötigt. Anhand der mittels der erfindungsgemäßen Messvorrichtung bestimmbaren Pulswellengeschwindigkeit sowie auch anhand der Blutdruckamplitude und des Pulses lässt sich die Reaktion des Körpers auf die Nahrungsaufnahme bestimmen. Hierzu ist zweckmäßigerweise die Auswertungseinheit der erfindungsgemäßen Messvorrichtung zur Auswertung des zeitlichen Verlaufs der Pulswellengeschwindigkeit und zur Ermittlung der Zusammensetzung von einem Benutzer der Messvorrichtung aufgenommener Nahrung anhand des zeitlichen Verlaufs der Pulswellengeschwindigkeit ab dem Zeitpunkt der Nahrungsaufnahme eingerichtet. Die Pulswellengeschwindigkeit, sowie auch die Blutdruckamplitude und der Puls ändern sich, sobald die Nahrungsaufnahme beginnt. Die Maxima und die jeweiligen Zeitpunkte der Maxima sind dabei beeinflusst durch die Nahrungszusammensetzung. Der Verlauf und die absolute Höhe von Pulswellengeschwindigkeit, Blutdruckamplitude und Puls können herangezogen werden, um mittels der Auswertungseinheit der erfindungsgemäßen Messvorrichtung die Zusammensetzung der aufgenommenen Nahrung zu bestimmen.

Der Metabolismus des menschlichen Körpers ist im Normalzustand, d. h. in Ruhe und in der so genannten thermoneutralen Zone, im Wesentlichen durch den Glukosehaushalt bestimmt. Daher kann die Glukosekonzentration in den Zellen des Körpergewebes in diesen Normalzustand als reine Funktion der Wärmeproduktion und des Sauerstoffverbrauchs beschrieben werden. Es gilt:

$$[Glu] = f_1(\Delta T, VO_2) \, ,$$

wobei [Glu] für die Glukosekonzentration steht. Die Wärmeproduktion $\Delta T$ kann mittels des Wärmesensors der erfindungsgemäßen Messvorrichtung z.B. aus der Differenz zwischen der arteriellen Temperatur und der Temperatur, welche die Hautoberfläche bei perfekter thermischer Isolierung erreichen würde, bestimmt werden ($\Delta T = T_\infty - T_{Arterie}$). $f_1 (\Delta T, VO_2)$ gibt die funktionale Abhängigkeit der Glukosekonzentration von der Wärmeproduktion und vom Sauerstoffverbrauch an. Der Sauerstoffverbrauch ergibt sich, wie oben beschrieben, aus dem Unterschied zwischen venöser und arterieller Sauerstoffsättigung und der Durchblutung. Zur Bestimmung der Glukosekonzentration während bzw. direkt nach der Nahrungsaufnahme muss jedoch ein Korrekturterm berücksichtigt werden, der den Anteil des Fettstoffwechsels am Energiehaushalt wiedergibt. Es gilt dann:

$$[\text{Glu}] = f_1(\Delta T, VO_2) + X * f_2(\Delta T, VO_2)$$

X ist ein Faktor, der nach der Nahrungsaufnahme negativ ist. Dabei hängt X von der Zusammensetzung der aufgenommenen Nahrung ab. Insbesondere ist X davon abhängig, in welchem Verhältnis Fett und Kohlenhydrate am Metabolismus beteiligt sind. Der Faktor X lässt sich, wie oben beschrieben, anhand des zeitlichen Verlaufs der Pulswellengeschwindigkeit bestimmen. X ist 0, wenn reine Kohlenhydrate oder direkt Glukose aufgenommen werden. Der Betrag von X steigt an, je größer der Anteil von Fett an der aufgenommenen Nahrung ist. Zur Bestimmung des Korrekturfaktors X aus dem zeitlichen Verlauf der Pulswellengeschwindigkeit, der Blutdruckamplitude und/oder des Pulses wird normalerweise eine Eichung der erfindungsgemäßen Messvorrichtung zur Anpassung an den jeweiligen Benutzer der Vorrichtung erforderlich sein. $f_2 (\Delta T, VO_2)$ gibt für den Fettstoffwechsel die funktionale Abhängigkeit der Glukosekonzentration von der Wärmeproduktion und vom Sauerstoffverbrauch an.

Die Auswertungseinheit der erfindungsgemäßen Messvorrichtung kann somit zur Bestimmung der lokalen Glukosekonzentration aus dem lokalen Sauerstoffverbrauch und der lokalen Wärmeproduktion eingerichtet sein. Hierzu muss die Messvorrichtung die geeigneten Messmodalitäten aufweisen. Die Ermittlung des Sauerstoffverbrauchs, kann, wie oben erläutert, durch die Kombination der Oximetrie mit der bioelektrischen Impedanzmessung erfolgen. Zur Ermittlung der Wärmeproduktion ist dann noch zusätzlich ein geeigneter Wärmesensor erforderlich. Um schließlich die Glukosekonzentration nach dem oben angegebenen funktionalen Zusammenhang berechnen zu können, sollte noch der Korrekturfaktor X, beispielsweise aus dem zeitlichen Verlauf der Pulswellengeschwindigkeit, ermittelt werden. Dies kann, wie ebenfalls oben erläutert, durch kombinierte Messung von EKG-Signalen und pulsoximetrischen Signalen erfolgen. Zur Bestimmung der Glukosekonzentration sind also zweckmäßigerweise in der erfindungsgemäßen Messvorrichtung eine bioelektrische Impedanzmesseinheit, ein Pulsoximeter, eine EKG-Einheit, sowie ein Wärmesensor kombiniert.

Die zuvor skizzierte Methode erlaubt zunächst nur eine Bestimmung der intrazellulären Glukosekonzentration. Mit der Blutglukosekonzentration besteht vereinfacht der folgende Zusammenhang:

$$[\text{Glu}]_{Zelle} = a + b * \ln (c * [\text{Glu}]_{Blut})$$

Die Konstanten a, b und c hängen von der individuellen Physiologie des Benutzers der Messvorrichtung ab. Somit kann die Auswertungseinheit der erfindungsgemäßen Messvorrichtung weiterhin eingerichtet sein zur Bestimmung des Blutglukosespiegels aus der lokalen Glukosekonzentration, wobei von der Physiologie des Benutzers der Messvorrichtung abhängige Parameter berücksichtigt werden müssen. Diese Parameter können durch entsprechende Eichung bestimmt werden, beispielsweise durch Vergleich mit in herkömmlicher Weise invasiv bestimmten Blutglukosewerten.

Sinnvoll ist es, wenigstens eine, möglichst jedoch alle Messmodalitäten der erfindungsgemäßen Messvorrichtung mit einem Tiefpass- und/oder einem 50/60 Hz Filter zur Befreiung von überlagerten elektromagnetischen Störungen auszustatten. Dies kann entweder durch eine geeignete elektronische Beschaltung (z.B. vor der Analog-/Digital-Wandlung) oder innerhalb der Messdatenverarbeitung mittels der Auswertungseinheit geschehen.

Besonders bevorzugt ist eine Ausgestaltung der erfindungsgemäßen Messvorrichtung, bei welcher sämtliche Messmodalitäten zu einer kompakten Sensoreinheit zusammengefasst sind, und zwar in der Weise, dass sämtliche Messungen im selben Bereich die Hautoberfläche des zu untersuchenden Patienten erfolgen. Auf diese Weise wird eine kompakte Sensoreinheit geschaffen, die eine Vielzahl von diagnostischen Messwerten liefert. Diese können einzeln oder in Kombination ausgewertet werden, um schnell und zuverlässig aussagekräftige Informationen zum Gesundheitszustand des untersuchten Patienten zu erhalten. Die kompakte Sensoreinheit kann als vollständig funktionsfähiges Teil kostengünstig in großen Stückzahlen vorgefertigt und in Diagnosegeräte verschiedenster Art integriert werden. Die eigentliche Messung kann besonders einfach und komfortabel durchgeführt werden. Hierzu wird lediglich die Oberfläche des Sensorgehäuses, wo sich z.B. die Elektroden der Impedanzmesseinheit und der EKG-Einheit befinden, mit der Haut im Bereich des zu untersuchenden Körpergewebes in Kontakt gebracht. Dies kann z.B. durch einfaches Auflegen eines Fingers des Patienten auf die Gehäuseoberfläche der Sensoreinheit erfolgen. Die Impedanzmessung und die EKG-Ableitung erfolgen dann gleichzeitig über die die Sensoreinheit berührende Hautstelle.

Die erfindungsgemäße Messvorrichtung kann in ein mobiles Monitoringgerät integriert sein, bei welchem die oben erwähnten medizinischen Messmodalitäten einzeln oder in Kombination die Datenaufnahmeeinheit zur Messdatenaufnahme bilden und bei welchem die programmgesteuerte Auswertungseinheit zur Auswertung der aufgenommenen Messdaten vorgesehen ist. Eine Speichereinheit dient zur Speicherung der aufgenommen und/oder der berechneten/ ausgewerteten Daten. Eine Anzeigeeinheit ist vorgesehen zur Visualisierung der aufgenommenen und/oder ausgewerteten Daten. Eine Datenübertragungseinheit dient dazu, die aufgenommenen Daten und/oder die berechneten/ausgewerteten Daten an externe Geräte zu übertragen. Hierbei kann es sich um eine übliche drahtgebundene oder auch um eine drahtlose Schnittstelle (beispielsweise nach dem Bluetooth-Standard) handeln. Die in der Speichereinheit des Monitoringgerätes abgespeicherten Daten können von einem behandelnden Arzt ausgelesen und ausgewertet werden, um den Verlauf von Behandlungen des Patienten zu überwachen. Die Datenübertragungsschnittstelle des Monitoringgerätes kann zur Übertragung der in der Speichereinheit des Monitoringgerät gespeicherten Daten an einen Personalcomputer des Arztes genutzt werden. Sinnvollerweise kann auch eine Datenfernübertragung der mittels der Sensoreinheit erfassten und ausgewerteten diagnostischen Daten erfolgen. Die Datenübertragung kann z.B. über ein Datennetz (Internet) erfolgen. Alternativ können die diagnostischen Daten über ein Mobilfunknetz übermittelt werden. Die rohen Messsignale oder die ausgewerteten diagnostischen Daten können zum Beispiel an eine zentrale Stelle ("Healthcare-Center") zur eingehenderen Analyse und Dokumentation sowie zur Überwachung der zeitlichen Entwicklung einzelner Werte übermittelt werden. Dort werden die Daten z.B. mittels geeigneter Analysealgorithmen ggf. unter Berücksichtung von hinterlegten Patientendaten (einschließlich Informationen bezüglich chronischer Erkrankungen oder Vorerkrankungen) ausgewertet. Das Ergebnis kann wiederum über das jeweilige Daten- oder Kommunikationsnetz zurück gesendet werden, um den Benutzer der Messvorrichtung entsprechend über seinen Gesundheitszustand zu informieren. Von der zentralen Stelle aus können auch bei Bedarf weitere gezielte Messungen mittels der erfindungsgemäßen Sensoreinheit initiiert werden. Außerdem können zum Zwecke einer erweiterten Anamnese veranlasst durch die Auswertungsergebnisse Rückfragen an den Patienten über das Daten- oder Kommunikationsnetz übermittelt werden. Falls sich aus den Mess- und Auswertungsergebnissen Hinweise auf einen medizinischen Notfall ergeben, können die erforderlichen Maßnahmen (z.B. automatische Alarmierung des Rettungsdienstes) sofort in die Wege geleitet werden. Ein weiterer Vorteil der Datenfernübertragung ist, dass die erforderliche Software zur Auswertung der Messsignale nicht in der Vorrichtung selbst implementiert sein muss, sondern lediglich an der zentralen Stelle, wo die Daten empfangen werden, vorgehalten und gepflegt werden muss.

Gemäß einer bevorzugten Ausführungsform der Messvorrichtung sind die Datenaufnahmeeinheit (die Impedanzmesseinheit), die Auswertungseinheit, die Speichereinheit, die Anzeigeeinheit und die Übertragungseinheit in einem gemeinsamen Gehäuse untergebracht. Damit ist die Vorrichtung kompakt aufgebaut und kann als mobiles Gerät jederzeit und überall benutzt werden. Die Messvorrichtung kann ein eigenständiges Gerät sein, oder es kann in ein auch anderweitig nutzbares elektronisches Gerät (z.B. Armbanduhr, Mobiltelefon, MP3-Player, Digital-Kamera) integriert sein. Denkbar ist auch die Verbindung der erfindungsgemäßen Messvorrichtung mit einem beliebigen Gerät der Unterhaltungs- oder Kommunikationstechnik, wie z.B. Desktop, Notebook, Laptop, Mobiltelefon, Palmtop oder Handheld. Der Nutzer eines solchen Geräte kann schnell, komfortabel und unauffällig jederzeit eine Messung zur Erfassung der interessierenden physiologischen Parameter durchführen. Aufgrund der geringen Größe der Sensorik der erfindungsgemäßen Messvorrichtung kann diese auch in ein beliebiges Accessoire, wie z.B. eine Brille, eine Armbanduhr, ein Schmuckstück oder dergleichen, oder in ein Kleidungsstück (sog. "Smart Clothes") integriert werden.

Gemäß einer weiteren bevorzugten Ausgestaltung der erfindungsgemäßen Messvorrichtung ist eine Fixiervorrichtung zur Fixierung eines Körperteils, z.B. eines Fingers, des zu untersuchenden Patienten vorgesehen. Bei Impedanzmessungen und auch bei pulsoximetrischen Messungen hat der Anpressdruck des Körpergewebes (z.B. des Fingers) auf den optischen Sensor bzw. auf die Mess- und Einspeiseelektroden der Impedanzmesseinheit signifikanten Einfluss auf die Messsignale. Demzufolge kann es sinnvoll sein, mittels der Fixiervorrichtung für einen definierten Anpressdruck zu sorgen. Die Fixiervorrichtung kann z.B. ein aufblasbares Luftkissen umfassen, welches das entsprechende Körperteil (sanft) gegen die Mess- und/oder Einspeiseelektroden bzw. gegen die optischen Sensoren presst und dort fixiert. Durch die Fixierung werden vorteilhaft auch Bewegungen des Körperteils unterbunden, die das Messergebnis verfälschen könnten.

Bei einer weiteren vorteilhaften Ausgestaltung der erfindungsgemäßen Messvorrichtung ist eine Mehrzahl von Einspeise- und/oder Messelektroden matrixförmig angeordnet. Dies ermöglicht es, unterschiedliche räumliche Konfigurationen bei der Wechselstromeinspeisung und bei der Spannungsmessung zu erzeugen. Die dabei gewonnenen zusätzlichen Informationen ermöglichen es, Rückschlüsse auf den pH-Wert, den $pCO_2$-Wert, den $pO_2$-Wert sowie auf den Elektrolythaushalt ($Na^+$-, $K^+$-, $Ca^{2+}$-, $Mg^{2++}$-Konzentration etc.) zu ziehen.

Ausführungsbeispiele der Erfindung werden im Folgenden anhand der Zeichnungen näher erläutert. Es zeigen:

Figur 1    Schematische Ansicht der Impedanzmesseinheit der erfindungsgemäßen Messvorrichtung;

Figur 2    Draufsicht auf eine erfindungsgemäße Messvorrichtung als kompakte Sensoreinheit;

Figur 3    Erfindungsgemäße Messvorrichtung mit kreisförmigen Mess- und Einspeiseelektroden;

Figur 4    Blockdiagramm der erfindungsgemäßen Messvorrichtung;

Figur 5    schematische Ansicht eines alternativen Ausführungsbeispiels der erfindungsgemäßen Messvorrichtung;

Figur 6    Blockschaltbild der Impedanzmesseinheit der erfindungsgemäßen Messvorrichtung.

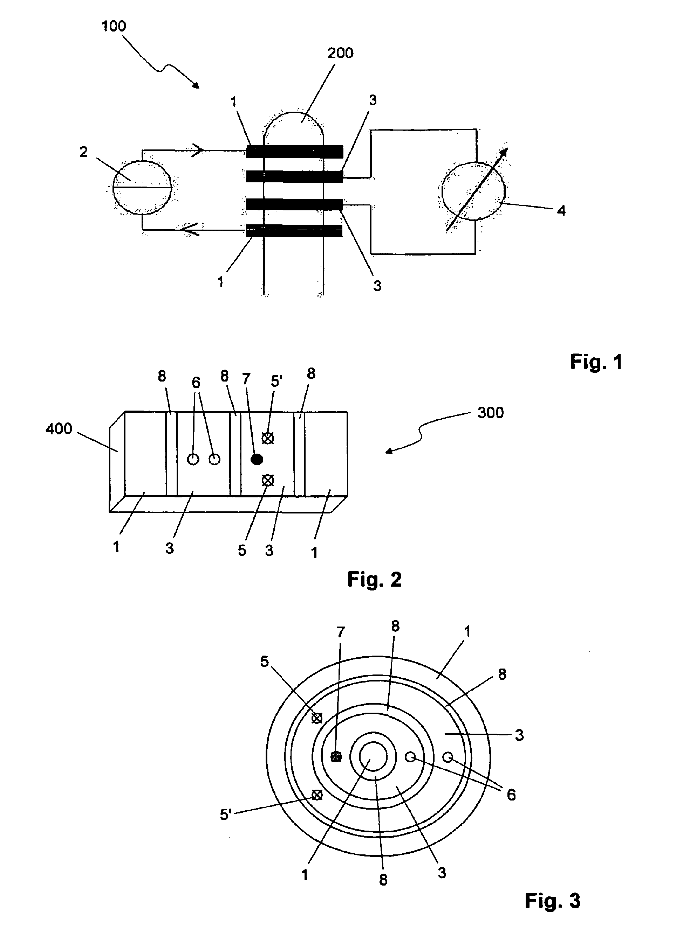

Die in der Figur 1 illustrierte bioelektrische Impedanzmesseinheit 100 der erfindungsgemäßen Messvorrichtung umfasst zur Bestimmung der lokalen Resistanz und der lokalen Reaktanz zwei Elektroden 1 zur Einspeisung von elektrischem Wechselstrom der Stromquelle 2 variabler Frequenz und zwei oder mehr Messelektroden 3 zur Impedanzmessung des Körpergewebes 200 im Bereich des Fingers des Benutzers der Vorrichtung. Aufgrund der Vierpunkt-Messung verfälschen Übergangswiderstände zwischen den Elektroden 1, 3 und dem Körpergewebe 200 die Messung nicht. Sinnvollerweise beträgt der Abstand zwischen den Elektroden 1, 3 nur einige Millimeter bis wenige Zentimeter. Beim Messvorgang berühren alle vier Elektroden 1, 3 zur lokalen Erfassung des Impedanzmesssignals gleichzeitig den selben Bereich der Hautoberfläche am Finger des Benutzers. Mittels der Stromquelle 2 wird ein Wechselstrom variabler Frequenz erzeugt. Auf diese Weise ist die Messung der komplexen Impedanz möglich. Das Messsignal wird mittels eines Spannungsmessers 4 erfasst. Sinnvollerweise wird das Messsignal mittels eines (in der Figur 1 nicht dargestellten) Analog-/Digitalwandlers digitalisiert und danach einer diskreten Fouriertransformation (DFT) unterzogen. Der DFT-Algorithmus liefert dann den Real- und den Imaginärteil der Impedanz, d.h. den Resistanz- und den Reaktanzwert. Die dargestellte Impedanzmesseinheit 100 kann, letztlich aufgrund des geringen Elektrodenabstands, sehr kompakt aufgebaut sein und somit gut in ein mobiles elektronisches Gerät (z.B. Armbanduhr, Mobiltelefon, MP3-Player, Digital-Kamera, Handheld usw.) integriert werden.

Die Figur 2 zeigt die insgesamt mit 300 bezeichnete Messvorrichtung gemäß der Erfindung als kompakte Sensoreinheit, die in ein beliebiges Gerät integrierbar ist. Die Messvorrichtung 300 weist verschiedene Messmodalitäten auf, die an der Oberfläche eines Sensorgehäuses 400 zugänglich sind. Die Abmessungen des Sensorgehäuses 400 betragen z.B. nur 10 x 7 x 3 mm. Diese berührt der Benutzer der Messvorrichtung 300 zur Durchführung einer Messung z.B. mit den Fingerspitzen. In die Messvorrichtung 300 sind Lichtquellen 5, 5' beispielsweise in Form von Leuchtdioden integriert, die dazu in der Lage ist, Licht bei verschiedenen Wellenlängen zu emittieren. Hierzu sind verschiedene lichtemittierende Halbleiterelemente in dem gemeinsamen Sensorgehäuse 400 untergebracht. Ebenso denkbar ist die Verwendung von Lichtwellenleitern, um das Licht von verschiedenen Lichtquellen an die Bedienoberfläche des Gehäuses 400 zu führen. Des Weiteren umfasst die Messvorrichtung 300 einen oder mehrere Fotosensoren 6. Die Fotosensoren sind in unmittelbarer Nähe zur Lichtquelle 5 bzw. 5' angeordnet. Die Sensoren 6 empfangen das im Gewebe an der Fingerspitze des Benutzers gestreute Licht der Lichtquelle 5 bzw. 5'. Weiterhin ist unmittelbar neben der Lichtquelle 5 bzw. 5' ein Wärmesensor 7 vorgesehen. Dadurch ist gewährleistet, dass die Wärmemessung am selben Messort erfolgt wie die optische Messung. Außerdem sind an der Oberfläche der Messvorrichtung 300 insgesamt vier Elektroden 1 bzw. 3 zur Messung der bioelektrischen Impedanz vorgesehen. Die Elektroden 1, 3 sind durch isolierende Streifen 8 voneinander getrennt. Der Benutzer der Vorrichtung berührt mit einer Hand gleichzeitig die vier Elektroden, d.h. die Mess- und Einspeiseelektroden, wie oben in Bezug auf die Figur 1 erläutert. Zumindest eine der mit der Bezugsziffer 1 bezeichneten Elektroden wird außerdem als EKG-Elektrode einer ebenfalls in die Messvorrichtung 300 integrierten EKG-Einheit verwendet. Eine weitere EKG-Elektrode (Gegenelektrode, nicht dargestellt) ist anderswo angeordnet, so dass die Elektroden mit den Fingerspitzen berührt werden können. Es ergibt sich eine Zweipunkt-Ableitung (Arm-zu-Arm-Messung).

Bei dem in der Figur 2 dargestellten Ausführungsbeispiel der Messvorrichtung 300 sind zwei Strahlungsquellen 5 und 5' vorgesehen, welche unterschiedliche Volumenbereiche des untersuchten Körpergewebes bestrahlen. Hierzu haben die zwei Strahlungsquellen 5 und 5' unterschiedliche räumliche Abstrahlcharakteristiken, nämlich unterschiedliche Abstrahlwinkel. Bei der Strahlungsquelle 5 handelt es sich um eine Leuchtdiode, während es sich bei der Strahlungsquelle 5' um einen Laser, beispielsweise einen so genannten VCSEL-Laser (engl. "vertical cavity surface emitting laser") handelt. Sowohl die Leuchtdiode 5 als auch der Laser 5' emittieren Licht mit sehr ähnlicher Wellenlänge (z. B. 630 nm und 650 nm), aber mit unterschiedlichen Öffnungswinkeln (z. B. 25° und 55°). Mit der in der Figur 2 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 5 und 5' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 2 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobektischer Punkt). Das von der Leuchtdiode bzw. von dem Laser emittierte Licht kann mittels entsprechender Lichtleitfasern an die entsprechende Stelle an der Gehäuseoberfläche der Tastatur geführt werden. In diesem Fall sind mit den Bezugsziffern 5 und 5' in der Figur 1 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 5 als auch der Strahlungsquelle 5' wird mittels der Sensoren 6 detektiert. Die Sensoren 6 müssen nicht direkt an der Oberfläche des Gehäuses 400 angeordnet sein. Stattdessen kann das Licht über Lichtleitfasern den im Inneren der Messvorrichtung 300 angeordneten Sensoren zugeführt werden. Zur Unterscheidung des Lichtes der Strahlungsquelle 5 von dem Licht der Strahlungsquelle 5' können die beiden Lichtquellen 5 und 5' unterschiedlich zeitlich moduliert betrieben werden, wobei die mittels der Sensoren 6 detektierten Signale entsprechend demoduliert werden. Alternativ ist es möglich, die Strahlung der beiden Strahlungsquellen 5 und 5' anhand der unterschiedlichen Wellenlänge zu unterscheiden. Die Strahlungsintensität der von den Strahlungsquellen 5 und 5' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 2 dargestellten Sensoren 6 können die interessierenden Parameter der Intensitätsschwächung bestimmt werden, und zwar getrennt für die von den Strahlungsquellen 5 und 5' jeweils erfassten Volumenbereiche des untersuchten Körpergewebes. Die den verschiedenen Strahlungsquellen 5 und 5' zuzuordnenden Parameter der Intensitätsschwächung können mittels einer geeignet programmgesteuerten Auswertungseinheit zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 5 und 5' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 5 und 5' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits vom Blutglukosespiegel abhängt, kann mittels der erläuterten differenziellen Messung der Strahlungsabsorption auch der Blutglukosespiegel ermittelt werden. Parallel zur optischen Messung wird die Bioimpedanzanalyse über die Elektroden 1, 3 durchgeführt. Zweck der Bioimpedanzmessung ist vor allem die Bestimmung der lokalen Durchblutung. Diese wird als weiterer Parameter bei der Bestimmung des Sauerstoffverbrauchs und damit auch des Blutglukosespiegels herangezogen. Unterschiedliche Öffnungswinkel der Strahlung können auch mit nur einer Strahlungsquellen 5 durch Verwendung entsprechender optischer Elemente (z.B. Strahlteiler, Linsen etc.) erzeugt werden.

Die Figur 3 zeigt eine alternative Ausgestaltung des erfindungsgemäßen Messvorrichtung 300 mit konzentrisch angeordneten, im Wesentlichen kreisförmigen Elektroden 1, 3. Je nach zu untersuchender Körperstelle ist die Anordnung gemäß Figur 2 oder diejenige gemäß Figur 3 besser geeignet.

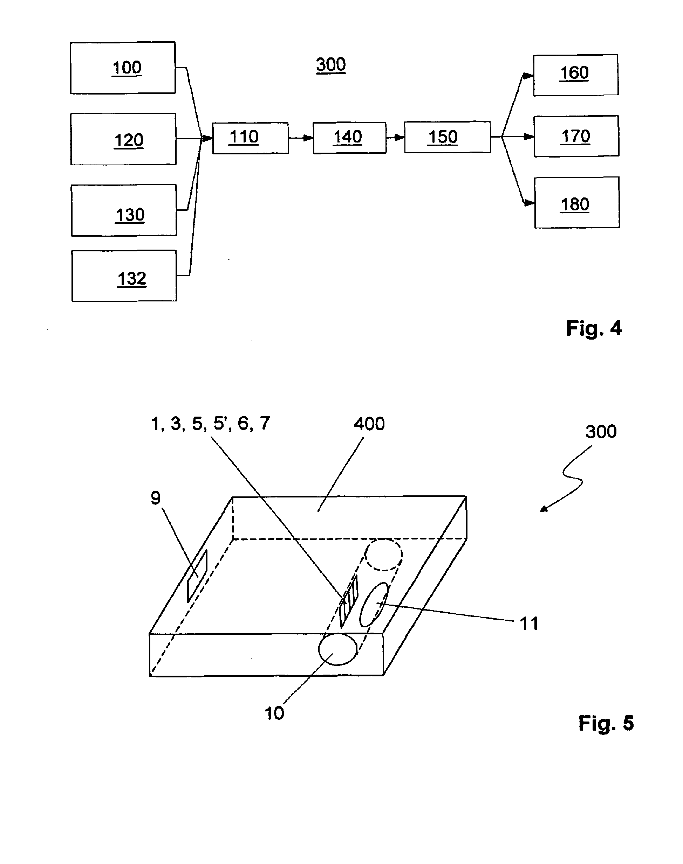

Die Figur 4 zeigt schematisch den Aufbau der erfindungsgemäßen Messvorrichtung 300 als Blockdiagramm. Messvorrichtung 300 umfasst eine optische Messeinheit 130 zur optischen Messung der Sauerstoffkonzentration im Blutgefäßsystem des Körpergewebes am jeweiligen Messort. Die mittels der optischen Messeinheit 130 erfassten pulsoximetrischen Signale werden einer Analyseeinheit 110 zugeführt. Eine weitere wesentliche Komponente der Messvorrichtung 300 ist eine Wärmemesseinheit 120 zur Bestimmung der lokalen Wärmeproduktion. Bei der Wärmemesseinheit 120 handelt es sich um einen speziellen Wärmesensor, welcher die jeweils untersuchte Körperstelle isoliert. Diese Stelle kann somit nur noch Wärme durch den Blutstrom aufnehmen oder abgeben. Daher ist es möglich, durch die zeitaufgelöste Messung der Temperatur die Durchblutung und die Wärmeproduktion zu bestimmen. Bei einer starken Durchblutung erreicht die untersuchte Körperstelle in sehr kurzer Zeit ihre maximale Temperatur. Bei geringer Durchblutung dauert dies länger. Zusätzlich kann über die Extrapolation der gemessenen Temperatur auf die arterielle Temperatur geschlossen werden, da die Temperatur am Ort der Messung nur durch die arterielle Temperatur und durch die lokale Wärmeproduktion bestimmt wird. Auch die mittels der Wärmemesseinheit 120 erfassten Messsignale werden der Analyseeinheit 110 zur Weiterverarbeitung zugeführt. Außerdem umfasst Messvorrichtung 300 die Impedanz-messeinheit 100, die zur Erfassung von lokalen Gewebeparametern mittels bioelektrischer Impedanzmessung dient. Die Messsignale der Impedanz-messeinheit 100 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 130 sind die Lichtquellen 5, 5' sowie die Lichtsensoren 6 der in den Figuren 2 und 3 dargestellten Messvorrichtung 300 zugeordnet. Die Wärmemesseinheit 120 ist mit dem Wärmesensor 7 verbunden. Die Impedanzmesseinheit 100 erfasst Messsignale über die Elektroden 1 bzw. 3 der Messvorrichtung 300. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 130, der Wärmemesseinheit 120, der Impedanz-Messeinheit 100 und der EKG-Einheit 132 in eine Auswertungseinheit 140. Die Auswertungseinheit 140 ist dafür zuständig, aus den Messsignalen die für die Diagnose wesentlichen Parameter zu berechnen. Die Funktionen der Auswertungseinheit 140 sind im Wesentlichen durch Software realisiert. Aus den zeitabhängig aufgenommenen Messsignalen der Impedanzmesseinheit 100 wird z.B. die Zusammensetzung des untersuchten Körpergewebes (Wassergehalt, Fettgehalt usw.) berechnet. Aus den Signalen der optischen Messeinheit 130 wird die arterielle Sauerstoffsättigung und - unter Zugrundelegung der zuvor auf der Basis der Impedanzmessung ermittelten Gewebeparameter - die kapillare Sauerstoffsättigung berechnet. Weiterhin werden aus den Messsignalen der Wärmemesseinheit 120 und aus den Daten, die aus der zeitabhängigen Impedanzmessung ableitbar sind, die Durchblutung und die arterielle Temperatur bestimmt. Aus den Signalen der EKG-Einheit 132 und denjenigen der optischen Messeinheit 130 wird die Pulswellengeschwindigkeit bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch und die Glukosekonzentration am Messort berechnet. Die Berechnungsergebnisse werden mittels einer Diagnoseeinheit 150 interpretiert. Die Diagnoseeinheit 150 dient zur Bewertung der mittels der Auswertungseinheit 140 berechneten lokalen metabolischen Parameter. Die Auswertungseinheit 140 und die Diagnoseeinheit 150 sind zur Anzeige der Messresultate mit einer Grafikeinheit 160 verbunden. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Übertragung der berechneten physiologischen Parameter dient. Über die Schnittstelleneinheit 180 können sämtliche Daten und Parameter, insbesondere auch die in der Speichereinheit 170 gespeicherten Daten und Parameter, z.B. an einen nicht näher dargestellten PC eines behandelnden Arztes übertragen werden. Dort können die Daten detaillierter analysiert werden. Insbesondere können über einen längeren Zeitraum mit der Messvorrichtung 300 aufgenommene Daten und Parameter auf Veränderungen hin untersucht werden, um daraus Schlussfolgerungen hinsichtlich der Entwicklung einer bestehenden Erkrankung ableiten zu können.

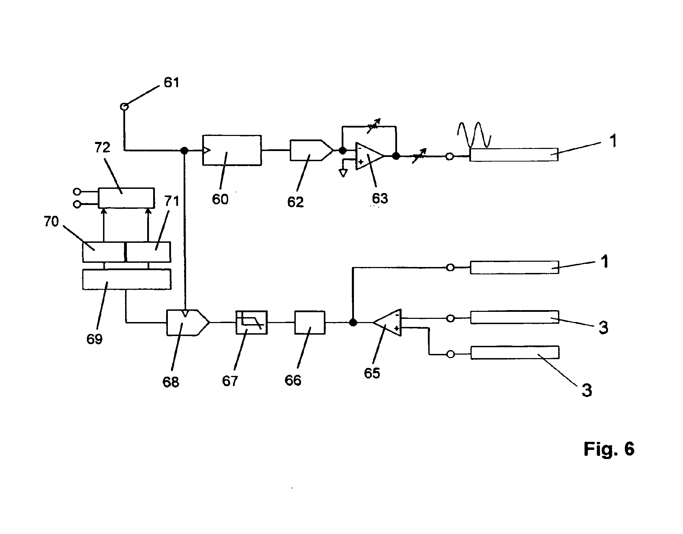

Die Figur 5 zeigt schematisch ein weiteres Ausführungsbeispiel der erfindungsgemäßen Messvorrichtung 300. An der Außenseite des Gehäuses 400 ist eine EKG-Elektrode 9 angebracht. Diese Elektrode wird mit dem Finger einer Hand berührt. Ein Finger der anderen Hand wird in eine röhrenförmige Öffnung 10 eingeführt. Im Inneren der Öffnung 10 befinden sich die Elektroden 1, 3, die Lichtquellen 5, 5', die Lichtsensoren 6 sowie der Wärmesensor 7. Weiterhin ist im Inneren der Röhre 10 ein aufblasbares Luftkissen 11 angeordnet, welches den Finger fixiert und sanft und mit definiertem Druck gegen die Sensoren presst. Bedientasten der Messvorrichtung 300 sowie eine Anzeige zur Ausgabe der Messergebnisse sind aus Gründen der Übersichtlichkeit in der Figur 6 weggelassen.

Die Figur 6 zeigt den schaltungstechnischen Aufbau der Impedanz-messeinheit 100 der erfindungsgemäßen Messvorrichtung 300 als Blockdiagramm. Die Impedanzmesseinheit 100 umfasst einen digitalen Signalgenerator 60, der mit einem externen Taktsignal 61 beaufschlagt wird. Das Digitalsignal wird mittels eines Digital-/Analogwandlers 62 in ein Analogsignal umgewandelt und mittels eines Verstärkers 63 verstärkt. Auf diese Weise wird ein Wechselstromsignal variabler Frequenz erzeugt, das über die Einspeiseelektrode 1 dem Körper des untersuchten Patienten zugeführt wird. Das Impedanzmesssignal wird über eine Messelektrode 3 erfasst und mittels eines Verstärkers 65 verstärkt. Dem Verstärker 65 sind ein variabler Abschwächer 66 und ein Tiefpassfilter 67 zum Zwecke der Rauschunterdrückung nachgeschaltet. Das verstärkte und gefilterte Analogsignal wird mittels eines Analog-/Digitalwandlers 68 in ein Digitalsignal umgewandelt und mittels einer digitalen Fouriertransformationseinheit 69 transformiert. Der Real- und Imaginärteil des fouriertransformierten Messsignals werden in Registern 70 bzw. 71 gespeichert. Die Register 70 und 71 sind über eine Schnittstelle 72 (z.B. I$^2$C-Schnittstelle) abfragbar. Über die Schnittstelle 72 werden die fouriertransformierten Digitalsignale an die Auswertungseinheit 140 der erfindungsgemäßen Messvorrichtung 300 übertragen.

**Patentansprüche**

1.  Medizinische Messvorrichtung mit einer Impedanz-messeinheit (100) zur Erfassung eines Impedanzmesssignals von der Hautoberfläche (200) eines zu untersuchenden Patienten über wenigstens ein Messelektrodenpaar (3), und mit einer mit der Impedanzmesseinheit (100) verbundene Auswertungseinheit (140), wobei der Elektrodenabstand des Messelektrodenpaares (3) weniger als einen Millimeter bis einige Zentimeter beträgt, in der Weise, dass beim Messvorgang beide Elektroden des Messelektrodenpaares (3) zur lokalen Erfassung des Impedanzmesssignals gleichzeitig im selben Bereich die Hautoberfläche (200) des Patienten berühren, **dadurch gekennzeichnet, dass** die Auswertungseinheit (140) zur Bestimmung von zeitlichen Änderungen des lokalen Impedanzmesssignals eingerichtet ist und dass die Auswertungseinheit (140) weiter eingerichtet ist zur Bestimmung

    - der Herzfrequenz,
    - der Pulsamplitude,
    - der lokalen Körpertemperatur und
    - der lokalen Durchblutung
    aus den zeitlichen Änderungen des lokalen Impedanzmesssignals.

**2.** Messvorrichtung nach Anspruch 1, **gekennzeichnet durch** ein Einspeiseelektrodenpaar (1) zur Aufprägung eines Wechselstroms variabler Frequenz über die Hautoberfläche (200) in das Körpergewebe des zu untersuchenden Patienten, und zwar in dem die Messelektroden (3) berührenden Bereich der Hautoberfläche (200).

**3.** Messvorrichtung nach Anspruch 1 oder 2, **dadurch gekennzeichnet, dass** die Mess- und Einspeiseelektroden (1, 3) als parallel zueinander verlaufende Kontaktstreifen ausgebildet sind.

**4.** Messvorrichtung nach einem der Ansprüche 1 bis 3, **dadurch gekennzeichnet, dass** die Auswertungseinheit (140) zur Bestimmung der lokalen Resistanz und der lokalen Reaktanz aus dem Impedanzmesssignal eingerichtet ist.

**5.** Messvorrichtung nach einem der Ansprüche 1 bis 4, **gekennzeichnet durch** eine EKG-Einheit (132) zur Erfassung eines EKG-Signals über zwei oder mehr EKG-Elektroden (3, 9).

**6.** Messvorrichtung nach Anspruch 5, **dadurch gekennzeichnet, dass** wenigstens eine der EKG-Elektroden (3, 9) gleichzeitig Einspeise- oder Messelektrode (1, 3) der bioelektrischen Impedanzmesseinheit (100) ist.

**7.** Messvorrichtung nach einem der Ansprüche 1 bis 6, **gekennzeichnet durch** eine optische Messeinheit (130), die wenigstens eine Strahlungsquelle (5) zur Bestrahlung des zu untersuchenden Körpergewebes und wenigstens einen Strahlungssensor (6) zur Detektion der von dem Körpergewebe gestreuten und/oder transmittierten Strahlung umfasst.

**8.** Messvorrichtung nach Anspruch 7, **dadurch gekennzeichnet, dass** die optische Messeinheit (130) wenigstens zwei Strahlungssensoren (6) zur Detektion der von dem Körpergewebe gestreuten und/oder transmittierten Strahlung aufweist, wobei die Strahlungssensoren (6) in unterschiedlichem Abstand zur Strahlungsquelle (5) angeordnet sind.

**9.** Messvorrichtung nach Anspruch 7 oder 8, **dadurch gekennzeichnet, dass** wenigstens zwei Strahlungsquellen (5, 5') vorgesehen sind, welche unterschiedliche Volumenbereiche des untersuchten Körpergewebes bestrahlen.

**10.** Messvorrichtung nach Anspruch 9, **dadurch gekennzeichnet, dass** die wenigstens zwei Strahlungsquellen (5, 5') unterschiedliche räumliche Abstrahlcharakteristiken haben.

**11.** Messvorrichtung nach einem der Ansprüche 7 bis 10, **dadurch gekennzeichnet, dass** die wenigstens eine Strahlungsquelle (5) mit einem lichtleitenden Element verbunden ist, welches die von der Strahlungsquelle (5) emittierte Strahlung zur Hautoberfläche (200) leitet.

**12.** Messvorrichtung nach einem der Ansprüche 1 bis 11, **gekennzeichnet durch** einen Temperatur- oder Wärmesensor (7).

**13.** Messvorrichtung nach einem der Ansprüche 1 bis 12, **gekennzeichnet durch** eine Fixiervorrichtung (11) zur Fixierung eines Körperteils des zu untersuchenden Patienten an der Messvorrichtung (300).

**14.** Messvorrichtung nach Anspruch 13, **dadurch gekennzeichnet, dass** die Fixiervorrichtung (11) ein aufblasbares Luftkissen umfasst, welches das Körperteil gegen die Mess- und/oder Einspeiseelektroden (1, 3) presst.

**15.** Messvorrichtung nach einem der Ansprüche 1 bis 14, **gekennzeichnet durch** eine Verbindung mit einem Gerät der Unterhaltungs- oder Kommunikationstechnik oder mit einem anderweitigen tragbaren Gerät oder Accessoire.

**16.** Messvorrichtung nach Anspruch 15, **dadurch gekennzeichnet, dass** das Gerät ein mobiles Gerät, insbesondere ein Notebook, ein Laptop, ein Mobiltelefon, ein Palmtop oder ein Handheld ist.

**17.** Messvorrichtung nach einem der Ansprüche 1 bis 16, **dadurch gekennzeichnet, dass** eine Mehrzahl von Einspeise- und/oder Messelektroden der Impedanzmesseinheit (100) matrixförmig angeordnet sind.

**Claims**

**1.** Medical measurement device having an impedance measurement unit (100) for detecting an impedance measurement signal on the skin surface (200) of a patient to be examined, by way of at least one measurement electrode pair (3), and having an evaluation unit (140) connected with the impedance measurement unit (100), wherein the distance between the electrodes of the measurement electrode pair (3) amounts to less than a millimeter up to a few centimeters, in such a manner that during the measurement process, the two electrodes of the measurement electrode pair (3) touch the skin surface (200) of the patient in the same region, at the same time, for local determination of the impedance measurement signal,

**characterized in that**

the evaluation unit (140) is set up for determining changes in the local impedance measurement signal and that the evaluation unit (140) is furthermore set up for determining

- the heart rate,
- the pulse amplitude,
- the local body temperature and
- the local perfusion

from the changes in the local impedance measurement signal over time.

2. Measurement device according to claim 1, **characterized by** a feed electrode pair (1) for imposing an alternating current of variable frequency, by way of the skin surface (200), into the body tissue of the patient to be examined, specifically in the region of the skin surface (200) that touches the measurement electrodes (3).

3. Measurement device according to one of claims 1 to 2, **characterized in that** the measurement and feed electrodes (1, 3) are configured as contact strips that run parallel to one another.

4. Measurement device according to one of claims 1 to 3, **characterized in that** the evaluation unit (140) is set up for determining the local resistance and the local reactance from the impedance measurement signal.

5. Measurement device according to one of claims 1 to 4, **characterized by** an EKG unit (132) for detecting an EKG signal by way of two or more EKG electrodes (3, 9).

6. Measurement device according to claim 5, **characterized in that** at least one of the EKG electrodes (3, 9) is simultaneously a feed electrode or measurement electrode (1, 3) of the bioelectrical impedance measurement unit (100).

7. Measurement device according to one of claims 1 to 6, **characterized by** an optical measurement unit (130) that comprises at least one radiation source (5) for irradiating the body tissue to be examined, and at least one radiation sensor (6) for detection of the radiation scattered and/or transmitted by the body tissue.

8. Measurement device according to claim 7, **characterized in that** the optical measurement unit (130) has at least two radiation sensors (6) for detection of the radiation scattered and/or transmitted by the body tissue, whereby the radiation sensors (6) are disposed at different distances from the radiation source (5).

9. Measurement device according to claim 7 or 8, **characterized in that** at least two radiation sources (5, 5') are provided, which irradiate different volume regions of the body tissue that is examined.

10. Measurement device according to claim 9, **characterized in that** the at least two radiation sources (5, 5') have different spatial emission characteristics.

11. Measurement device according to one of claims 7 to 10, **characterized in that** the at least one radiation source (5) is connected with a light-conducting element that guides the radiation emitted by the radiation source (5) to the skin surface (200).

12. Measurement device according to one of claims 1 to 11, **characterized by** a temperature or heat sensor (7).

13. Measurement device according to one of claims 1 to 12, **characterized by** a fixation device (11) for fixation of a body part of the patient to be examined, on the measurement device (300).

14. Measurement device according to claim 13, **characterized in that** the fixation device (11) comprises an inflatable air cushion that presses the body part against the measurement and/or feed electrodes (1, 3).

15. Measurement device according to one of claims 1 to 14, **characterized by** a connection with a device of entertainment or communications technology or with some other portable device or accessory.

16. Measurement device according to claim 15, **characterized in that** the device is a mobile device, particularly a notebook, a laptop, a mobile telephone, a palmtop, or a handheld.

17. Measurement device according to one of claims 1 to 16, **characterized in that** a plurality of feed and/or measurement electrodes of the impedance measurement unit are disposed in the form of a matrix.

## Revendications

1. Dispositif de mesure médical avec une unité de mesure d'impédance (100) pour la détection d'un signal de mesure d'impédance de la surface de la peau (200) d'un patient à examiner par au moins une paire d'électrodes de mesure (3), et une unité d'évaluation (140) reliée à l'unité de mesure d'impédance (100), où l'espacement entre les deux électrodes de mesure (3) représente moins qu'un millimètre jusqu'à quelques centimètres, de telle manière que lors de l'opération de mesure, les deux électrodes de la paire d'électrodes de mesure (3), pour la détection locale du signal de mesure d'impédance, viennent simultanément en contact, dans la même zone, avec la surface de la peau (200) du patient, **caractérisé en ce que** l'unité d'évaluation (140) est agencée pour la détermination de modifications dans le temps du signal de mesure d'impédance local, et **en ce que** l'unité d'évaluation (140) est agencée en outre pour la détermination

   - de la fréquence cardiaque,
   - de l'amplitude du pouls,
   - de la température locale du corps et
   - de la circulation locale
   à partir des modifications dans le temps du signal de mesure d'impédance local.

2. Dispositif de mesure selon la revendication 1, **caractérisé par** une paire d'électrodes d'alimentation (1) pour appliquer un courant alternatif d'une fréquence variable par la surface de la peau (200) dans le tissu corporel du patient à examiner, à savoir dans la zone de la surface de la peau (200) venant en contact avec les électrodes de mesure (3).

3. Dispositif de mesure selon la revendication 1 ou 2, **caractérisé en ce que** les électrodes de mesure et d'alimentation (1, 3) sont réalisées comme bandes de contact s'étendant parallèlement l'une à l'autre.

4. Dispositif de mesure selon l'une des revendications 1 à 3, **caractérisé en ce que** l'unité d'évaluation (140) est agencée pour la détermination de la résistance locale et de la réactance locale à partir du signal de mesure d'impédance.

5. Dispositif de mesure selon l'une des revendications 1 à 4, **caractérisé par** une unité d'électrocardiogramme (132) pour la détection d'un signal d'électrocardiogramme par deux électrodes ECG ou plus (3, 9).

6. Dispositif de mesure selon la revendication 5, **caractérisé en ce qu'**au moins une des électrodes ECG (3, 9) est en même temps l'électrode d'alimentation ou de mesure (1, 3) de l'unité de mesure d'impédance bioélectrique (100).

7. Dispositif de mesure selon l'une des revendications 1 à 6, **caractérisé par** une unité de mesure optique (130) qui comprend au moins une source de rayonnement (5) pour l'irradiation du tissu corporel à examiner et au moins un capteur de rayonnement (6) pour la détection du rayonnement dispersé et/ou transmis par le tissu corporel.

8. Dispositif de mesure selon la revendication 7, **caractérisé en ce que** l'unité de mesure optique (130) présente au moins deux capteurs de rayonnement (6) pour la détection du rayonnement dispersé et/ou transmis par le tissu corporel, où les capteurs de rayonnement (6) sont disposés à des écarts différents de la source de rayonnement (5).

9. Dispositif de mesure selon la revendication 7 ou 8, **caractérisé en ce qu'**au moins deux sources de rayonnement (5, 5') sont prévues qui irradient les zones de volume différentes du tissu corporel à examiner.

**10.** Dispositif de mesure selon la revendication 9, **caractérisé en ce que** les au moins deux sources de rayonnement (5, 5') ont des caractéristiques de rayonnement spatiales différentes.

**11.** Dispositif de mesure selon l'une des revendications 7 à 10, **caractérisé en ce que** la au moins une source de rayonnement (5) est reliée à un élément conducteur de lumière qui conduit le rayonnement émis par la source de rayonnement (5) à la surface de la peau (200).

**12.** Dispositif de mesure selon l'une des revendications 1 à 11, **caractérisé par** un capteur de température ou de chaleur (7).

**13.** Dispositif de mesure selon l'une des revendications 1 à 12, **caractérisé par** un dispositif de fixation (11) pour la fixation d'une partie corporelle du patient à examiner au dispositif de mesure (300).

**14.** Dispositif de mesure selon la revendication 13, **caractérisé en ce que** le dispositif de fixation (11) comprend un coussin d'air gonflable qui presse la partie de corps contre les électrodes de mesure et/ou d'alimentation (1, 3).

**15.** Dispositif de mesure selon l'une des revendications 1 à 14, **caractérisé par** une liaison avec un appareil de la technique de divertissement ou de communication ou avec un autre appareil ou accessoire portable.

**16.** Dispositif de mesure selon la revendication 15, **caractérisé en ce que** l'appareil est un appareil mobile, en particulier un ordinateur bloc-notes, un ordinateur portable, un téléphone mobile ou un ordinateur de poche.

**17.** Dispositif de mesure selon l'une des revendications 1 à 16, **caractérisé en ce que** plusieurs électrodes d'alimentation et/ou de mesure de l'unité de mesure d'impédance (100) sont disposées en forme de matrice.

**Fig. 1**

**Fig. 2**

**Fig. 3**

| 100 |
| 120 |
| 130 |
| 132 |

300

| 110 | → | 140 | → | 150 |

| 160 |
| 170 |
| 180 |

**Fig. 4**

1, 3, 5, 5', 6, 7    400                    300

9

11

10

**Fig. 5**

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **MEIR NITZAN ; BORIS KHANOKH.** Infrared Radiometry of Thermally Insulated Skin for the Assessment of Skin Blood Flow. *Optical Engineering,* 1994, vol. 33 (9), 2953-2956